# EUROPÄISCHE PATENTSCHRIFT

(11) **EP 1 785 093 B1**
(45) Veröffentlichungstag und Bekanntmachung des Hinweises auf die Patenterteilung: **03.06.2009**
(21) Anmeldenummer: 06123421.7
(22) Anmeldetag: 03.11.2006
(51) Int. Cl.: A61B 6/00, A61B 6/04, A61N 5/10, A61B 6/03, A61B 5/055

(54) **Bildgebungsvorrichtung und Therapieanlage mit einer solchen Bildgebungsvorrichtung**
Imaging apparatus and therapy system incorporating such an imaging apparatus
Dispositif d'imagerie et système de thérapie équipé d'un tel dispositif

(30) Priorität: 09.11.2005 DE 102005053488
(43) Veröffentlichungstag der Anmeldung: 16.05.2007
(73) Patentinhaber: SIEMENS AKTIENGESELLSCHAFT, 80333 München (DE)
(72) Erfinder: Rietzel, Eike, 64289, Darmstadt (DE); Sommer, Andres, 91094, Langensendelbach (DE)

(56) Entgegenhaltungen:
- EP-A- 1 238 685
- EP-A- 1 629 774
- EP-A1- 0 832 603
- CH-A5- 691 655
- DE-A1- 19 703 556

## Beschreibung

Die Erfindung bezieht sich auf eine Bildgebungsvorrichtung zur Erstellung von digitalen Bilddaten eines Körperbereichs eines Patienten. Die Erfindung bezieht sich weiterhin auf eine Therapieanlage mit einer solchen Bildgebungsvorrichtung.

Für die Therapieplanung, insbesondere im Rahmen einer Strahlentherapie, werden üblicherweise dreidimensionale Bilddaten des Patientenkörpers herangezogen, um den zu behandelnden Körperbereich, z.B. einen Tumor, im Vorfeld der eigentlichen Therapie möglichst exakt zu lokalisieren. Diese Bilddaten werden üblicherweise in einem jeder Behandlungssitzung vorausgehenden Bildgebungsschritt aufgenommen, insbesondere um gegebenenfalls eine räumliche Veränderung dieses Körperbereichs gegenüber früheren Behandlungen, z.B. eine Größenänderung oder Verschiebung eines Tumors im Körpergewebe zu erkennen. Zur Erzeugung des dreidimensionalen Bilddatensatzes werden übliche medizinische Bildgebungsverfahren, insbesondere Computertomographie (CT), Magnetresonanztomographie (MR), Positronen-Emissions-Tomographie (PET) oder Einzelphotonen-Emissions-Computertomographie (single photon emission computer tomography, kurz: SPECT) herangezogen. Der Begriff Strahlentherapie umfasst im Rahmen der folgenden Ausführungen sowohl Strahlentherapie im engeren Sinne, bei welcher hochenergetische elektromagnetische Strahlung auf Tumorgewebe appliziert wird, als auch Partikeltherapieverfahren, bei welchen Tumorgewebe mit Teilchenstrahlung (z.B. beschleunigten Protonen, Carbonionen, etc.) beschossen wird.

Die Maschineneinstellungen der Bestrahlungsvorrichtung - also z.B. Bestrahlungsrichtung, Position, Feldgröße usw. - werden üblicherweise relativ in Bezug auf ein vorgegebenes Patientenisozentrum im Patientenkörper festgelegt, das den Ursprung eines Patientenkoordinatensystems bildet. Dieser Punkt kann zum Beispiel in die Mitte eines auf den dreidimensionalen Bilddaten sichtbaren Tumorvolumens gelegt werden.

Es ist nun üblich, mit Hilfe elektronisch gesteuerter Laserstrahlen Linien auf die Haut des Patienten zu projizieren, die die Lage dieses Punktes markieren. Diese Linien werden mit Hilfe von Farbmarkierungen auf der Haut eingezeichnet und müssen bis zum nächsten Untersuchungstermin oder zum Bestrahlungstermin sichtbar bleiben. Falls nötig muss der Patient für ein Nachzeichnen der Linien einbestellt werden.

Bei einer aus CH 691 655 A5 bekannten Bildgebungsvorrichtung werden mittels Tomographie aufgenommene Schnittbilder eines bewegungslos auf einer Auflagefläche liegenden Körpers unabhängig von dem Körper referenziert. Dazu wird in einem Kalibrationsschritt zumindest die Lage eines in der Bildaufnahmeebene der Bildgebungsvorrichtung liegenden Punktes in einem raumfesten Koordinatensystem bestimmt. Während eines Bildaufnahmeschrittes wird jedem Schnittbild die Lage der Aufnahmefläche so zugeordnet, dass diese Lage auch in raumfesten Koordinaten bestimmbar ist.

Aus DE 197 03 556 A1 ist eine weitere Bildgebungsvorrichtung mit einer Bildaufnahmeeinheit in Form eines C-Bogen-Röntgengeräts bekannt. Bei dieser Vorrichtung wird mittels eines 3D-Kamerasystems an der Bildaufnahmeeinheit die dreidimensionale Position von Leuchtmarkierungen an einem Patiententisch bestimmt. Anhand dieser Information werden Positionen, die in einem Detektorkoordinatensystem bestimmt wurden, in Objektkoordinaten eines tischfesten Objektkoordinatensystems umgerechnet.

Diese Aufgabe wird bezüglich einer Bildgebungsvorrichtung, wie sie für eine Therapieplanung verwendbar ist, erfindungsgemäß gelöst durch die Merkmale des Anspruchs 1. Bezüglich einer Therapieanlage mit einer solchen Bildgebungsvorrichtung sowie einer Therapievorrichtung wird die Aufgabe erfindungsgemäß gelöst durch die Merkmale des Anspruchs 6. Bevorzugte oder vorteilhafte Ausführungsformen sind durch die Unteransprüche oder die nachfolgende Beschreibung gegeben.

Die Bildgebungsvorrichtung umfasst einen Tisch zur Lagerung eines Patienten (nachfolgend als Bildgebungstisch bezeichnet) sowie eine Bildaufnahmeeinheit zur Erstellung von digitalen Bilddaten, die einen Bereich des Körperinneren des Patienten abbilden. Erfindungsgemäß ist hierbei vorgesehen, dass die Bildaufnahmeeinheit die Koordinaten der aufgenommenen Bilddaten nicht (lediglich) in Bezug auf ein Patientenkoordinatensystem skaliert, sondern (zumindest auch) in Bezug auf ein Raumkoordinatensystem, in Bezug auf welches der Bildgebungstisch ortsfest montiert ist. Zusätzlich oder alternativ ist erfindungsgemäß vorgesehen, dass die Koordinaten der Bilddaten in Bezug auf ein Tischkoordinatensystem räumlich skaliert werden, das ortsfest bezüglich einer Patientenauflage, d.h. der "Tischplatte" des Bildgebungstischs, definiert ist.

Der Begriff "skalieren" wird hierbei wörtlich im Sinne von "mit einer (räumlichen) Skala versehen" verwendet. Im Zuge der "Skalierung" wird dem Bilddatensatz also eine Lage- oder Ortsinformation zugeordnet, die jedem Bildpunkt des Bilddatensatzes einen Punkt innerhalb des Raum- bzw. Tischkoordinatensystems, und somit einen Raumpunkt zuweist. Die Skalierung bezieht sich somit auf die Koordinaten der Bildpunkte innerhalb des Bilddatensatzes. Die Skalierung beinhaltet dagegen keine Veränderung der Größe, d.h. der Bildpunktanzahl, des Bilddatensatzes.

Der Begriff "Raumkoordinatensystem" umfasst gemäß obiger Definition jedes Koordinatensystem, in dem der Bildgebungstisch ortsfest montiert ist, das also mit mindestens einem Befestigungspunkt des Bildgebungstischs in einer konstanten räumlichen Beziehung steht. Bevorzugt ist das Raumkoordinatensystem auch ortsfest bezüglich des umgebenden Bildgebungsraums definiert, und der Bildgebungstisch entsprechend ortsfest gegenüber dem umgebenden Bildgebungsraum montiert. Jedoch kann im Sinne einer kinematischen Umkehr auch eine Beweglichkeit des Bildgebungstisches gegenüber dem umgebenden Bildgebungsraum vorgesehen sein, wobei in diesem Fall das Raumkoordinatensystem mit dem Bildgebungstisch mitbewegt wird.

Das Raumkoordinatensystem und das Tischkoordinatensystem bilden im Wesentlichen äquivalente bzw. redundante Bezugssysteme zur Beschreibung der Lage eines Raumpunktes in dem Bildgebungsraum, von denen im Rahmen der Erfindung wahlweise eines oder beide explizit definiert sein können. Das Tischkoordinatensystem und das Raumkoordinatensystem stehen - zumindest solange die (optional abnehmbare) Patientenauflage mit dem Bildgebungstisch verbunden ist - in einer definierten räumlichen Beziehung und sind ineinander überführbar.

Durch die Skalierung der Koordinaten der Bilddaten in Bezug auf das Raumkoordinatensystem bzw. das Tischkoordinatensystem wird erreicht, dass die während einer Messung erzeugten Bilddaten einen absoluten Bezug zu einem Raumbereich aufweisen, der wiederum in einer definierten räumlichen Beziehung zu der Bildgebungsvorrichtung steht. Infolge dieser definierten räumlichen Beziehung können Aufnahmen auf besonders einfache und präzise Weise identisch oder im wesentlichen identisch reproduziert werden. Insbesondere kann hierdurch die Lage eines zu behandelnden Körperbereichs unabhängig von Markierungen an oder im Körper des Patienten gekennzeichnet und das invasive Einbringen implantierter Markierungen (sog. BBs) in Tumornähe und die damit verbundenen Risiken für den Patienten vermieden werden.

Bevorzugt ist die Bildgebungsvorrichtung als Röntgensystem, insbesondere Computertomograph ausgebildet. Alternativ hierzu kann die Bildgebungsvorrichtung auch auf Basis einer anderen Bildgebungstechnik, insbesondere MR, PET oder SPECT, ausgebildet sein. Die digitalen Bilddaten enthalten insbesondere eine dreidimensionale Bildinformation, bilden also einen 3D-Bilddatensatz oder Volumendatensatz. Die Erfindung wird optional aber auch auf zweidimensionale Bilddaten angewendet.

Die Patientenauflage des Bildgebungstischs ist zur immobilisierten und im Wesentlichen reproduzierbaren Aufnahme des Patienten vorgesehen. Die Patientenauflage ist dabei insbesondere in Form einer Liege ausgebildet, die zur Immobilisierung des Patienten z.B. mit einer individuell an den Patienten angepassten geschäumten Kunststoffschale oder sonstigen einstellbaren Patientenfixierungsorganen ausgestattet ist. Alternativ hierzu kann die Patientenauflage aber auch nach Art eines Stuhls ausgebildet sein, so dass der Patient in sitzender Haltung gelagert ist.

Die Patientenauflage ist vorzugsweise von einem fest montierten Stativ des Bildgebungstisches reversibel abnehmbar. Dies hat den Vorteil, dass der Patient in immobilisiertem Zustand weiteren Behandlungsschritten zugeführt werden kann und ermöglicht somit sowohl einen zeitsparenden Behandlungsablauf als auch eine besonders präzise Therapieplanung, zumal die Körperhaltung des Patienten nach der Lokalisierung des Patientenisozentrums nicht mehr verändert werden muss. Diese Ausführungsform birgt weiterhin den Vorteil, dass für jeden Patienten auf einfache Weise eine individuell angepasste Patientenauflagevorrichtung an die Bildgebungsvorrichtung angekoppelt werden kann.

Um bezüglich der Platzierung der Patientenauflage auf dem Stativ reproduzierbare Bedingungen zu schaffen, ist bevorzugt vorgesehen, dass die Patientenauflage nur in einer definierten Position auf dem Stativ befestigbar ist.

Das Stativ ist insbesondere verstellbar, so dass die Patientenauflage insbesondere in der Höhe, jedoch optional auch horizontal gegenüber dem Raumkoordinatensystem versetzt werden kann. Das Stativ ist hierzu entweder manuell verstellbar oder als Roboterarm ausgebildet.

Die Bildaufnahmeeinheit der Bildgebungsvorrichtung ist gegenüber dem Bildgebungstisch, und damit gegenüber dem Raumkoordinatensystem, bewegbar, insbesondere auf Schienen oder dergleichen in einer definierten Bewegungsrichtung verschiebbar. Durch Kalibrierung einer Bewegungssteuerung der Bildaufnahmeeinheit, durch Sensoren oder auf andere Weise wird hierbei während der Bildaufnahme die Position der Bildaufnahmeeinheit in dem Raumkoordinatensystem erfasst und unter Heranziehung von hinterlegter Information über die Geometrie der Bildaufnahmeeinheit als Eingangsgröße für die Skalierung der Koordinaten der Bilddaten verwendet.

Die erfindungsgemäße Therapieanlage umfasst zusätzlich zu der der vorstehend beschriebenen Bildgebungsvorrichtung eine Therapievorrichtung, die insbesondere eine Bestrahlungseinheit zur Strahlenbehandlung des Patienten mit hochenergetischen elektromagnetischen Strahlen oder beschleunigten Partikeln umfasst. Der Therapievorrichtung ist ein weiteres Raumkoordinatensystem zugeordnet, in welchem ein Tisch zur Lagerung des Patienten (im Folgenden als Therapietisch bezeichnet) ortfest angeordnet ist. Zusätzlich oder alternativ ist der Therapievorrichtung ein weiteres Tischkoordinatensystem zugeordnet, das ortsfest bezüglich einer Patientenauflage des Therapietischs angeordnet ist.

Die Bildgebungsvorrichtung und die Therapievorrichtung sind hierbei durch entsprechende Kalibrierung bzw. Justierung derart aufeinander abgestimmt, dass anhand der Skalierung, d.h. der Lageinformation der Koordinaten der von der Bildgebungsvorrichtung aufgenommenen Bilddaten sowie einer hinterlegten Umrechnungsvorschrift ein entsprechender Raumbereich in dem Raum- bzw. Tischkoordinatensystem der Therapievorrichtung bestimmbar ist. Mit anderen Worten dient die Umrechnungsvorschrift zur Übertragung des in den Bilddaten abgebildeten Raumvolumens des Bildgebungsraums auf ein entsprechendes Raumvolumen des Therapieraums. Somit ist mit Hilfe der Umrechnungsvorschrift aus den skalierten Bilddaten bestimmbar, an welchem Raumpunkt in der Umgebung der Therapievorrichtung ein in den Bilddaten abgebildeter Körperbereich des Patienten, insbesondere ein Tumor, angeordnet ist, wenn der Patient auf dem Therapietisch gelagert wird. Die skalierten Bilddaten können direkt für eine Therapieplanung herangezogen werden.

In einer besonders bevorzugten Ausführung der Erfindung sind die Tische der Bildgebungs- und Therapievorrichtung gleichartig aufgebaut und eingestellt sowie bezüglich des jeweiligen Raumkoordinatensystems gleich angeordnet. Bei dieser Ausführung reduziert sich die Umrechungsvorschrift auf einen Faktor 1, d.h. die Lageinformation der Bilddaten gibt identisch die Lage des abgebildeten Körperbereichs des in der Therapievorrichtung gelagerten Patienten in dem zugehörigen Raum- bzw. Tischkoordinatensystem wieder.

Sind die Tische der Bildgebungs- bzw. Therapievorrichtung verschieden aufgebaut oder eingestellt oder bezüglich des jeweiligen Raumkoordinatensystems unterschiedlich angeordnet, so ist die zwischen den beiden Raumkoordinatensystemen vermittelnde Umrechungsvorschrift von 1 verschieden und anhand der örtlichen Gegebenheiten im Einzelfall zu bestimmen. Beträgt beispielsweise die Tischhöhe des Therapietisches das Doppelte der Tischhöhe der Bildgebungstisches, so wird die Skalierung der Koordinaten der Bilddaten durch die Umrechungsvorschrift hinsichtlich der Höhe um einen Faktor 2 korrigiert, etc.

Der Therapietisch weist insbesondere eine abnehmbare Patientenauflage und eine mechanische Schnittstelle zum Ankoppeln einer Patientenauflage auf, wobei die Patientenauflage und die Schnittstelle kompatibel zu den entsprechenden Teilen des Bildgebungstischs ausgebildet sind. Die gleichartige Ausbildung der beiden Tische ermöglicht einen Austausch der Patientenauflage zwischen der Bildgebungsvorrichtung und der Therapievorrichtung, und damit insbesondere einen Transport des Patienten in immobilisiertem Zustand.

Nachfolgend wird ein Ausführungsbeispiel der Erfindung anhand von Zeichnungen näher erläutert. Dabei zeigen:
- FIG 1: in schematischer Seitenansicht eine Bildgebungsvorrichtung mit einer Bildaufnahmeeinheit und einem Tisch zur Lagerung und Positionierung eines Patienten und
- FIG 2: in Blockdarstellung eine Therapieanlage mit einer Bildgebungsvorrichtung gemäß FIG 1 und einer Therapievorrichtung.

Einander entsprechende Teile sind in allen Figuren stets mit den selben Bezugzeichen versehen.

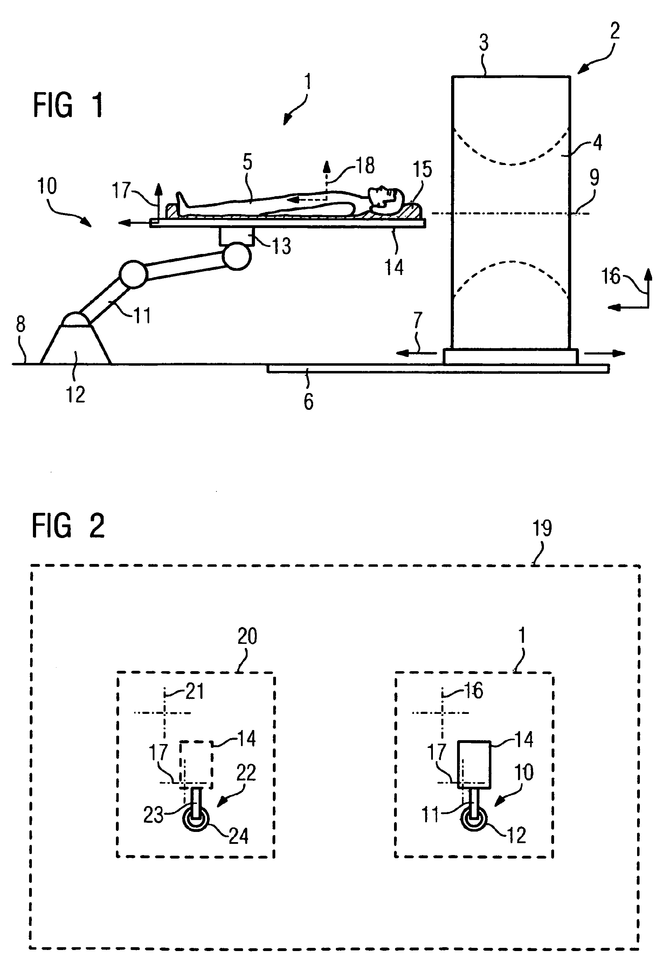

Die FIG 1 zeigt eine Bildgebungsvorrichtung 1. Die Bildgebungsvorrichtung 1 umfasst als Bildaufnahmeeinheit einen Computertomographen 2 (kurz CT). Der Computertomograph 2 umfasst eine CT-Gantry 3 mit einem Kanal 4, in den ein Patient 5 während einer computertomographischen Untersuchung eingeschoben wird. Die CT-Gantry 2 ist auf einem Schienensystem 6 entlang einer Verschieberichtung 7 verschiebbar auf dem Boden 8 eines Bildgebungsraums angeordnet und fixiert. Die Verschieberichtung 7 ist horizontal und damit insbesondere parallel zu einer isozentrischen Achse 9 der CT-Gantry 3 ausgerichtet, entlang welcher oder parallel zu welcher in der Regel auch der Patient 5 bezüglich seiner Körperlängsachse ausgerichtet ist. Der Patient 5 kann davon abweichend aber auch geneigt zu der isozentrischen Achse 9 gelagert sein.

Die Bildgebungsvorrichtung 1 umfasst weiterhin einen Tisch 10 zur Lagerung und Positionierung des Patienten 5 in der Untersuchungsposition. Der Tisch 10 umfasst ein Stativ 11, welches als manuell verstellbares Stativ oder als Roboterarm ausgebildet und mit einem Stativfuß 12 ortsfest auf dem Boden 8 fixiert ist. An einem von dem Stativfuß 12 beabstandeten Ende des Stativs 11 ist mechanische Schnittstelle 13 angeordnet, mittels welcher eine Patientenauflage 14 abnehmbar in einer definierten Position an dem Stativ 11 gehaltert ist. Die Patientenauflage 14 ist nach Art einer Patientenliege ausgebildet. Die Patientenauflage 14 weist ferner Fixierungsorgane 15 auf, die den Patienten 5 auf der Patientenauflage 14 immobilisiert in der vorgesehenen Untersuchungsstellung halten. Die Fixierorgane umfassen z.B. eine an den Patienten 5 individuell angepasste Schale oder Hohlform aus geschäumten Kunststoff, in die der Patientenkörper oder einzelne Körperteile desselben für die Untersuchung eingebettet werden.

Bei einer CT-Untersuchung fährt die CT-Gantry 3 über das Schienensystem 6 entlang der Verschieberichtung 7, wobei der Kanal 4 über den auf dem Tisch 10 angeordnete Patient 5 geführt wird, so dass der Patient 5 relativ bezüglich der CT-Gantry 3 durch den Kanal 4 bewegt wird.

Bei der Bildgebungsvorrichtung 1 ist zum einen ein Raumkoordinatensystem 16 definiert, das ortsfest in dem Bildgebungsraum angeordnet ist, und in welchem der Tisch 10 entsprechend mit seinem Stativfuß 12 feststehend montiert ist. Zum anderen ist ein Tischkoordinatensystem 17 in Bezug auf die Patientenauflage 14 definiert. Zwischen dem Raumkoordinatensystem 16 und dem Tischkoordinatensystem 17 besteht - solange die Patientenauflage 14 ihrerseits über die Schnittstelle 13 an das Stativ 11 gekoppelt ist - eine definierte räumliche Beziehung, die nur von der Stativeinstellung abhängt.

Ein drittes Koordinatensystem, nachfolgend als Patientenkoordinatensystem 18 bezeichnet, wird herkömmlicherweise in Bezug auf den Patientenkörper definiert. Anders als nach herkömmlicher Technik stellt das Patientenkoordinatensystem 18 im Rahmen der vorliegenden Erfindung eine reine Hilfsgröße dar, die nur optional und zum Zweck der Verdeutlichung konkret festgelegt wird. Der Ursprung des Patientenkoordinatensystems 18 wird üblicherweise in das Zentrum eines zu behandelnden Körperbereichs des Patienten 5 gelegt, der beispielsweise einen oder mehrere Tumore enthält. Das Patientenkoordinatensystem 18 charakterisiert insbesondere die Position dieser Tumore innerhalb des Patientenkörpers. Im Folgenden wird stellvertretend für den zu behandelnden Körperbereich verallgemeinernd auf das Patientenkoordinatensystem 18 und dessen Ursprung (nachfolgend als Patientenisozentrum bezeichnet) Bezug genommen.

Im Rahmen der Erfindung werden die Koordinaten der mittels der Bildgebungsvorrichtung 1 aufgenommenen Bilddaten nicht (oder zumindest nicht ausschließlich) in Bezug auf das Patientenkoordinatensystem 18, sondern in Bezug auf das Raumkoordinatensystem 16 skaliert. Ziel ist, die absolute Lage des Patientenisozentrums in den Raumkoordinaten zu ermitteln und diese in einen Therapieraum zu übertragen.

Hierzu wird durch den Computertomographen 2 während der Bildaufnahme die Verfahrstellung der CT-Gantry 3 auf dem Schienensystem 6 erfasst. Anhand von hinterlegter Information über die Ausrichtung des Schienensystems 6 im Bildgebungsraum berechnet der Computertomograph hieraus die Stellung der CT-Gantry 3 in Bezug auf das Raumkoordinatensystem 16. Anhand von weiterer hinterlegter Information über die Geometrie der CT-Gantry 3 (insbesondere über Höhe und Lage der isozentrischen Achse 9 im Bildgebungsraum) wird hieraus die Lage der aufgenommenen 3D-Bilddaten in Bezug auf das Raumkoordinatensystem 16 bestimmt. Anhand dieser Lageinformation werden die Koordinaten der Bilddaten skaliert, d.h. die ermittelte Lageinformation wird zusammen mit den 3D-Bilddaten abgelegt, so dass eine Zuordnung der Bilddaten zu den aufgenommenen Raumvolumen, bezogen auf das Raumkoordinatensystem 16, möglich ist.

Die derart skalierten Bilddaten enthalten somit die Information über die absolute Position des Patientenisozentrums bezüglich des Raumkoordinatensystems 16, und damit bezüglich des Bildgebungsraums. Wird die Stativeinstellung geändert, so wird die damit verbundene Verlagerung des Patientenisozentrum im Raum durch eine hinterlegte Korrekturfunktion in Abhängigkeit der Stativverstellung berücksichtigt. Ebenso wird, wenn die Patientenauflage 14 mit dem darauf immobilisierten Patienten 5 von dem Stativ 11 der Bildgebungsvorrichtung 1 abgenommen und auf dem Stativ eines weiteren Bildgebungs- oder Therapieraumes befestigt wird, die absolute Lage des Patientenisozentrums in dem neuen Bildgebungs- oder Therapieraum durch eine hinterlegte Umrechnungsvorschrift (die beispielsweise einen Größenunterschied der beiden Stative wiedergibt) berücksichtigt. Insbesondere kann die Lageinformation der 3D-Bilddaten aber identisch übernommen werden, wenn beide Stative einander in ihrem geometrischen Aufbau, der Stativeinstellung und ihrer Stellung bezüglich eines jeweils zugeordneten Raumkoordinatensystems gleichen.

Zusätzlich oder alternativ werden die Koordinaten der 3D-Bilddaten in Bezug auf das Tischkoordinatensystem 17 skaliert. Hierzu wird während der Bildaufnahme auch die Einstellung des Stativs 11 erfasst, und hieraus unter Rückgriff auf hinterlegte Information über die Position des Stativfußes 12 im Bildgebungsraum und über die Geometrie des Stativs 11 die räumliche Beziehung zwischen dem Raumkoordinatensystem 16 und dem Tischkoordinatensystem 17 bestimmt. Unter Nutzung dieser räumlichen Beziehung wird die Lageinformation der aufgenommenen 3D-Bilddaten mathematisch von dem Raumkoordinatensystem 16 in das Tischkoordinatensystem 17 transformiert.

Durch die Skalierung der Koordinaten der 3D-Bilddaten in Einheiten des Tischkoordinatensystems 17 ist die Lage des Patientenisozentrums ausschließlich in Bezug auf die Patientenauflage 14 definiert. Dies hat den Vorteil, dass diese Lageinformation auch dann noch direkt verwertbar ist, wenn die Patientenauflage 14 von dem Stativ 11 abgenommen wird.

Die FIG 2 zeigt eine Therapieanlage 19 für eine Strahlentherapie. Die Therapieanlage 19 umfasst (mindestens) einen Bildgebungsraum, in dem die vorstehend beschriebene Bildgebungsvorrichtung 1 angeordnet ist, sowie (mindestens) einen Therapieraum mit einer Therapievorrichtung 20, die eine (nicht näher dargestellte) Bestrahlungseinheit umfasst. Der Therapievorrichtung 20 ist ein weiteres Raumkoordinatensystem 21 zugeordnet, dessen Ursprung insbesondere in den Strahlengang eines (nicht näher dargestellten) Therapiestrahls gelegt ist. Bei einem rotierbaren Therapiestrahl (Bestrahlungsgantry) ist der Ursprung des Raumkoordinatensystems insbesondere in das Isozentrum des Therapiestrahls, gelegt.

Die Therapievorrichtung 20 umfasst des Weiteren einen Tisch 22 mit einem Stativ 23, das identisch zu dem Stativ 11 des Tisches 10 der Bildgebungsvorrichtung 1 aufgebaut ist. Das Stativ 23 ist bevorzugt mit seinem Stativfuß 24 in gleicher Position zu dem Raumkoordinatensystem 21 montiert wie das Stativ 11 der Bildgebungsvorrichtung 1 bezüglich des Raumkoordinatensystems 16. Die Stative 11 und 23 können aber auch bezüglich des jeweiligen Raumkoordinatensystems 16 bzw. 21 unterschiedlich angeordnet sein. In diesem Fall wird die unterschiedliche Tischanordnung durch eine hinterlegte, empirisch festgelegte Korrekturvorschrift berücksichtigt.

Die Tische 10 und 22 sind mechanisch gleichartig ausgebildet. Sie sind insbesondere auch mit gleichartigen Patientenauflagen 14 ausgestattet bzw. können die selbe Patientenauflage 14 austauschsweise aufnehmen.

Im Zuge einer Strahlentherapiesitzung wird der Patient 5 zunächst in der Bildgebungsvorrichtung 1 untersucht. Hierbei wird zur Planung der nachfolgenden Bestrahlung durch Aufnahme der Bilddaten insbesondere die Lage und Ausdehnung der zu behandelnden Tumore, Metastasen, etc. in Bezug auf das Raumkoordinatensystem 16 und/oder das Tischkoordinatensystem 17 festgestellt.

In einem nachfolgenden Schritt wird die Patientenauflage 14 mit dem darauf immobilisierten Patienten 5 von dem Stativ 11 abgenommen, zu der Therapievorrichtung 20 verbracht und dort mit dem Stativ 23 des Tischs 22 gekoppelt.

Durch die Kenntnis des geometrischen Aufbaus der Tische 10 und 22, ihrer Anordnung im jeweiligen Raumkoordinatensystem 16 bzw. 21 sowie der jeweiligen Stativstellung kann die absolute Position des Patientenisozentrums im Therapieraum, bezogen auf das Raumkoordinatensystem 21, durch eine hinterlegte Umrechnungsvorschrift ermittelt werden. Sind die Tische 10 und 22 gleich aufgebaut, angeordnet und eingestellt, so liegt der Patient 5 insbesondere in der Bildgebungsvorrichtung 1 bzw. in der Therapievorrichtung 20 in gleicher Lage relativ zu dem Raumkoordinatensystem 16 bzw. 21 des jeweiligen Bildgebungs- bzw. Therapieraumes.

Ist die Position des Patientenisozentrums im Raumkoordinatensystem 21 auf vorstehend beschriebene Weise bestimmt, so kann der Patient 5 durch Verstellung des Stativs 23 in die Bestrahlungsposition verfahren werden, wobei die Verlagerung des Patientenisozentraums im Raumkoordinatensystem 21 während der Stativverstellung durch eine hinterlegte Korrekturfunktion berücksichtigt wird. Die Bestrahlungsposition ist insbesondere derart bestimmt, dass das Patientenisozentrum im Ursprung des Raumkoordinatensystems 21, und damit im Strahlengang des Therapiestrahls, insbesondere in dessen Isozentrum liegt.

Die während der Untersuchung von der Bildgebungsvorrichtung 1 aufgenommenen und auf das Raumkoordinatensystem 16 skalierten 3D-Bilddaten können folglich daher unmittelbar zur Therapieplanung herangezogen werden. Das Positionieren von Markierungen in oder auf dem Patienten 5 ist aufgrund des absoluten Raumbezugs der 3D-Bilddaten nicht notwendig.

## Patentansprüche

1. Bildgebungsvorrichtung (1) mit einem Tisch (10) zur Lagerung eines Patienten (5) sowie mit einer Bildaufnahmeeinheit (2) zur Erstellung von digitalen Bilddaten eines Körperbereiches des Patienten (5), wobei die Bildaufnahmeeinheit (2) dazu ausgebildet ist, die Koordinaten der aufgenommenen Bilddaten in Bezug auf ein mit dem Tisch (10) ortsfestes Raumkoordinatensystem (16) und/oder in Bezug auf ein mit einer Patientenauflage (14) des Tischs (10) ortsfestes Tischkoordinatensystem (17) räumlich zu skalieren,
**dadurch gekennzeichnet, dass** die Bildaufnahmeeinheit (2) gegenüber dem Raumkoordinatensystem (16) bewegbar ist, wobei die Bildgebungsvorrichtung (1) dazu ausgebildet ist, während der Bildaufnahme als Eingangsgröße für die Skalierung der Koordinaten der Bilddaten die Position der Bildaufnahmeeinheit (2) in Bezug auf das Raumkoordinatensystem (16) zu erfassen.

2. Bildgebungsvorrichtung (1) nach Anspruch 1,
**dadurch gekennzeichnet, dass** die Bildgebungsvorrichtung (1) als CT, MR, PET, SPECT oder dergleichen ausgebildet ist, wobei die digitalen Bilddaten eine dreidimensionale Bildinformation enthalten.

3. Bildgebungsvorrichtung (1) nach Anspruch 1 oder 2,
**dadurch gekennzeichnet, dass** der Tisch (10) eine Patientenauflage (14) zur Lagerung und Positionierung des Patienten (5) sowie ein die Patientenauflage (14) halterndes Stativ (11) umfasst, wobei das Stativ (11) mit einem Stativfuß (12) ortsfest bezüglich des Raumkoordinatensystems (16) montiert ist.

4. Bildgebungsvorrichtung (1) nach Anspruch 3
**dadurch gekennzeichnet, dass** die Patientenauflage (14) reversibel abnehmbar in einer definierten Stellung an dem Stativ (11) befestigt ist.

5. Bildgebungsvorrichtung (1) nach Anspruch 3 oder 4,
**dadurch gekennzeichnet, dass** das Stativ (11) verstellbar ist.

6. Therapieanlage (19) mit einer Bildgebungsvorrichtung (1) nach einem der vorhergehenden Ansprüche sowie mit einer Therapievorrichtung (20) zur Durchführung einer Strahlentherapie, wobei die Therapievorrichtung (20) einen Tisch (22) zur Lagerung des Patienten (5) umfasst, der ortsfest in Bezug auf ein der Therapievorrichtung (20) zugeordnetes Raumkoordinatensystem (21) angeordnet ist und/oder bezüglich dessen Patientenauflage (14) ein Tischkoordinatensystem (17) definiert ist, wobei die Bildgebungsvorrichtung (1) und die Therapievorrichtung (20) derart kalibriert bzw. justiert sind, dass anhand einer vorgegebenen Umrechnungsvorschrift aus der mittels der Bildgebungsvorrichtung (1) ermittelten Skalierung der Koordinaten der Bilddaten ein diesen Bilddaten in dem Raumkoordinatensystem (21) bzw. Tischkoordinatensystem (17) der Therapievorrichtung (20) entsprechender Raumbereich bestimmt ist.

7. Therapieanlage (19) nach Anspruch 6,
**dadurch gekennzeichnet, dass** der Tisch (22) der Therapievorrichtung (20) gleichartig mit dem Tisch (10) der Bildgebungsvorrichtung (1) ausgebildet ist.

8. Therapieanlage (19) nach Anspruch 6 oder 7,
**dadurch gekennzeichnet, dass** der Tisch (22) der Therapievorrichtung (20) zu dem der Therapievorrichtung (20) zugeordneten Raumkoordinatensystem (21) in gleicher Weise ortsfest angeordnet ist, wie der Tisch (10) der Bildgebungsvorrichtung (1) zu dem dieser zugeordneten Raumkoordinatensystem (16).

## Claims

1. Imaging apparatus (1) with a table (10) for supporting a patient (5) and with an image recording unit (2) for creating digital image data of a body region of the patient (5), the image recording unit (2) being configured to scale the coordinates of the recorded image data spatially in respect of a spatial coordinate system (16) locationally fixed with the table (10) and/or in respect of a table coordinate system (17) locationally fixed with a patient support (14) of the table (10),
**characterised in that** the image recording unit (2) can be moved in relation to the spatial coordinate system (16), the imaging apparatus (1) being configured to capture the position of the image recording unit (2) in respect of the spatial coordinate system (16) as an input variable for scaling the coordinates of the image data during image recording.

2. Imaging apparatus (1) according to claim 1,
**characterised in that** the imaging apparatus (1) is configured as CT, MR, PET, SPECT or the like, the digital image data containing three-dimensional image information.

3. Imaging apparatus (1) according to claim 1 or 2,
**characterised in that** the table (10) comprises a patient support (14) for supporting and positioning the patient (5) and a stand (11) that supports the patient support (14), the stand (11) being mounted by means of a stand base (12) in a locationally fixed manner in respect of the spatial coordinate system (16).

4. Imaging apparatus (1) according to claim 3,
**characterised in that** the patient support (14) can be removed in a reversible manner in a defined position on the stand (11).

5. Imaging apparatus (1) according to claim 3 or 4,
**characterised in that** the stand (11) is adjustable.

6. Therapy system (19) with an imaging apparatus (1) according to one of the preceding claims and with a therapy apparatus (20) for performing radiation therapy, the therapy apparatus (20) comprising a table (22) for supporting the patient (5), the table (22) being disposed in a locationally fixed manner in respect of a spatial coordinate system (21) assigned to the therapy apparatus (20) and/or a table coordinate system (17) being defined in respect of its patient support (14), with the imaging apparatus (1) and the therapy apparatus (20) being calibrated or adjusted in such a manner that on the basis of a predetermined conversion code a spatial region corresponding to image data in the spatial coordinate system (21) or table coordinate system (17) of the therapy apparatus (20) is determined from the scaling of the coordinates of the image data determined by means of the imaging apparatus (1).

7. Therapy system (19) according to claim 6,
**characterised in that** the table (22) of the therapy apparatus (20) is configured in an identical manner to the table (10) of the imaging apparatus (1).

8. Therapy system (19) according to claim 6 or 7,
**characterised in that** the table (22) of the therapy apparatus (20) is disposed in a locationally fixed manner in relation to the spatial coordinate system (21) assigned to the therapy apparatus (20) in the same manner as the table (10) of the imaging apparatus (1) to the spatial coordinate system (16) assigned to it.

## Revendications

1. Dispositif d'imagerie (1) comprenant une table (10) pour l'installation d'un patient (5) ainsi qu'une unité d'enregistrement d'images (2) pour la constitution de données d'image numériques d'une zone du corps du patient (5), l'unité d'enregistrement d'images (2) étant exécutée pour réaliser une mise à l'échelle spatiale des coordonnées des données d'image enregistrées par rapport à un système de coordonnées spatial (16) fixe avec la table et/ou par rapport à un système de coordonnées de table (17) fixe avec un support de patient (14) de la table (10),
**caractérisé en ce que** l'unité d'enregistrement d'images (2) est mobile par rapport au système de coordonnées spatial (16), le dispositif d'imagerie (1) étant exécuté pour saisir la position de l'unité d'enregistrement d'images (2) par rapport au système de coordonnées spatial (16) en tant que grandeur d'entrée pour la mise à l'échelle des coordonnées des données d'image pendant l'enregistrement des images.

2. Dispositif d'imagerie (1) selon la revendication 1, **caractérisé en ce que** le dispositif d'imagerie (1) est exécuté en tant que CT, MR, PET, SPECT ou similaire, les données d'image numériques contenant une information d'image tridimensionnelle.

3. Dispositif d'imagerie (1) selon la revendication 1 ou 2, **caractérisé en ce que** la table (10) comprend un support de patient (14) pour l'installation et le positionnement du patient (5) ainsi qu'un statif (11) supportant le support de patient (14), le statif (11) étant monté fixement par rapport au système de coordonnées spatial (16) au moyen d'un pied de statif (12).

4. Dispositif d'imagerie (1) selon la revendication 3, **caractérisé en ce que** le support de patient (14) est fixé dans une position définie sur le statif (11) d'une manière amovible réversible.

5. Dispositif d'imagerie (1) selon la revendication 3 ou 4, **caractérisé en ce que** le statif (11) est réglable.

6. Système de thérapie (19) comprenant un dispositif d'imagerie (1) selon l'une des revendications précédentes ainsi qu'un dispositif de thérapie (20) pour l'exécution d'une radiothérapie, le dispositif de thérapie (20) comprenant une table (22) pour l'installation du patient (5) qui est fixe par rapport à un système de coordonnées spatial (21) associé au dispositif de thérapie (20) et/ou un système de coordonnées de table (17) étant défini par rapport au support de patient (14) de la table, le dispositif d'imagerie (1) et le dispositif de thérapie (20) étant calibrés resp. réglés de manière à ce que, partant de la mise à l'échelle des coordonnées des données d'image déterminée au moyen du dispositif d'imagerie (1), une zone spatiale correspondant à ces données d'image dans le système de coordonnées spatial (21) resp. système de coordonnées de table (17) du dispositif de thérapie (20) soit déterminée grâce à une règle de conversion prédéfinie.

7. Système de thérapie (19) selon la revendication 6, **caractérisé en ce que** la table (22) du dispositif de thérapie (20) est exécutée de manière identique à la table (10) du dispositif d'imagerie (1).

8. Système de thérapie (19) selon la revendication 6 ou 7, **caractérisé en ce que** la table (22) du dispositif de thérapie (20) occupe une même position fixe par rapport au système de coordonnées spatial (21) associé au dispositif de thérapie (20) que la table (10) du dispositif d'imagerie (1) par rapport au système de coordonnées spatial (16) associé à celui-ci.
